Europäisches Patentamt

(19) European Patent Office

Office européen des brevets

(11) Numéro de publication: **0 206 208**
**B1**

(12) **FASCICULE DE BREVET EUROPEEN**

(45) Date de publication du fascicule du brevet: **11.04.90**

(51) Int. Cl.⁵: **A 61 F 13/15**

(21) Numéro de dépôt: **86108207.1**

(22) Date de dépôt: **16.06.86**

(54) **Couche-culotte à jeter munie d'une ceinture élastique.**

(30) Priorité: **19.06.85 FR 8509345**

(43) Date de publication de la demande:
**30.12.86 Bulletin 86/52**

(45) Mention de la délivrance du brevet:
**11.04.90 Bulletin 90/15**

(84) Etats contractants désignés:
**AT BE CH DE GB IT LI NL**

(56) Documents cités:
**EP-A-0 119 825**
**FR-A-2 087 346**
**FR-A-2 289 131**
**FR-A-2 534 781**

(73) Titulaire: **PEAUDOUCE**
**59, Rue de la Vignette**
**F-59126 Linselles (FR)**

(72) Inventeur: **Courtray, Frank**
**110ter Rue de la Vignette**
**F-59126 Linselles (FR)**

(74) Mandataire: **Casalonga, Axel et al**
**BUREAU D.A. CASALONGA - JOSSE**
**Morassistrasse 8**
**D-8000 München 5 (DE)**

## Description

La présente invention a pour objet une couche-culotte à jeter pour enfants en bas âge ainsi qu'éventuellement pour adultes incontinents.

La présente invention a également pour objet certains procédés de fabrication de telles couches-culottes à jeter.

On sait que les couches-culottes qui sont utilisées généralement pour les enfants en bas âge mais aussi pour les adultes incontinents, comprennent sous une forme intégrée à titre d'éléments principaux, une enveloppe extérieure imperméable mince et souple jouant le rôle de culotte lorsque la couche-culotte est refermée et un matelas absorbant fixé à l'intérieur de l'enveloppe imperméable, recouvert d'un voile interne perméable à l'humidité réalisé par exemple en non-tissé. Des dispositifs d'attaches par adhésif sont en outre prévus sur les bords latéraux de la partie arrière de la couche-culotte afin de refermer cette dernière sur l'enfant au moment de l'utilisation. Une telle couche-culotte que l'on appelle parfois change-complet permet de remplacer à la fois la couche absorbante traditionnelle et la culotte imperméable qui était utilisée en combinaison avec ladite couche. On a déjà prévu également de disposer des éléments élastiques de chaque côté des bords longitudinaux du matelas absorbant ou au voisinage des échancrures permettant le passage des jambes lorsque la couche-culotte est portée. A titre d'exemples de telles couches-culottes munies d'élastiques à l'entrejambe on peut citer le brevet américain n° 3 860 003 et les brevets français n° 2 063 794, n° 2 438 434.

Les élastiques utilisés sont posés en continu à l'état tendu sur la majeure partie de leur longueur sur la face interne de la feuille imperméable. Leur fixation se fait en général par collage par exemple au moyen d'un adhésif à haute température qui est rapidement figé lorsque la température s'abaisse de façon à être immédiatement fixé en position tendue sur la majeure partie de sa longueur sur la face interne de la feuille imperméable souple. Ces élastiques sont habituellement utilisés sous la forme de bandelettes minces dont l'une des faces reçoit par intermittence un matériau adhésif à température élevée en fusion. On a également décrit dans le brevet français n° 2 539 274 un procédé permettant la fixation sur une enveloppe mince d'une multitude de brins élastiques individuels en latex naturel.

Ces éléments élastiques sont ensuite découpés en même temps que l'ensemble formé en continu et comprenant la feuille imperméable souple, les différents matelas absorbants et le voile perméable interne. Cette opération de découpe permet la rétraction de la portion des élastiques qui n'est pas collée sur la feuille imperméable de sorte que l'on obtient finalement une couche-culotte dont seules les zones d'entrejambe sont munies d'éléments élastiques, ce qui améliore la tenue de la couche-culotte en respectant parfaitement l'anatomie de l'utilisateur. La couche-culotte reste ainsi parfaitement ajustée à l'enfant malgré les mouvements de ce dernier.

Une difficulté subsiste cependant dans les couches-culottes de ce type couramment employées jusqu'à présent. Malgré l'existence des moyens élastiques d'entrejambe et la présence des attaches adhésives latérales on constate en effet que la couche-culotte n'est en général pas convenablement ajustée à la ceinture. Lorsque les adhésifs sont posés normalement on constate fréquemment que la couche-culotte baille à la fois sur le devant et sur le derrière de la zone de ceinture dès que l'enfant à bougé pendant un certain temps. De plus, il n'est pas possible d'ajuster correctement les attaches adhésives au moment de la pose.

L'amélioration de l'étanchéité à l'endroit de la ceinture des couches-culottes de ce type peut être obtenue en particulier par repli des bords extrêmes transversaux avant et arrière de la couche-culotte avant utilisation. Des moyens permettant de faciliter ce repli sont décrits par exemple dans les demandes de brevet français n° 84 00 180 et n° 84 00 179. Toutefois ces moyens ne remplissent pas pleinement leur rôle s'il n'est pas possible d'obtenir un ajustement convenable de la couche-culotte à l'endroit de la ceinture. Des culottes fermées ont été réalisées complètement à l'aide d'un matériau élastique (brevet français n° 2 529 056) ou prévues avec un élément élastique entourant complètement la ceinture (brevet français n° 2 490 079).

La demande de brevet français n° 2 515 004 décrit l'utilisation d'un organe d'étanchéité élastique fixé sur toute la largeur d'une couche-culotte et jouant également un rôle de fermeture grâce à des extensions recouvertes d'adhésif.

On a déjà songé également (demandes de brevet européen n° 119 827 et n° 119 825) à poser sur le bord extrême transversal des parties arrière et avant d'une couche-culotte, un matériau rétractable à chaud et présentant des caractéristiques d'élasticité à froid, tel que des polyuréthanes ou des compositions à base de mélanges d'élastomères et de résines thermoplastiques dont les utilisations sont décrites à titre général dans les brevet U.S. n° 3 912 565, n° 3 819 401 ou n° 4 303 571. Dans les demandes de brevet européen ci-dessus mentionnées, on pose une bande large transversalement au défilement de la feuille imperméable lors de sa fabrication. Cette bande transversale est réalisée en un matériau élastomère rétractable à chaud comme mentionné précédemment. La pose peut donc se faire dans l'état étiré du matériau, par collage au moyen de lignes longitudinales d'adhésif. On découpe ensuite les couches-culottes, la ligne de découpe passant sensiblement dans l'axe de la bande de matériau élastomère. Il en résulte que la couche-culotte finalement obtenue présente sur sa partie avant et sur sa partie arrière des bords extrêmes munis, après activation du matériau élastomère, d'une pluralité de canaux tubulaires ou fronces ouvertes vers l'extérieur. Des attaches adhésives classiques qui permettent la fermeture de la couche-culotte sont placées quant à elles dans une zone plus éloignée du bord transversal de la partie arrière de la couche-culotte. Il en résulte qu'une traction sur les attaches adhésives ne permet pas d'obtenir un ajustement convenable de la couche-culotte. De plus l'étanchéité au voisinage de la ceinture n'est pas conservée en raison de l'existence des fronces formant des canaux tubulaires qui sont incapables de retenir des fuites éventuelles de liquide.

La présente invention a pour objet de résoudre ces difficultés et de permettre la réalisation d'une couche-culotte qui puisse être convenablement ajustée de manière élastique dans la zone de la ceinture et parfaitement adaptée à la morphologie de l'utilisateur sans que l'étanchéité dans la zone de la ceinture soit en quelque manière que ce soit diminuée.

L'invention a encore pour objet de permettre la réalisation d'une couche-culotte de structure simplifiée permettant une économie sensible de matière tout en facilitant l'ajustement élastique mentionné préalablement.

La couche-culotte à jeter pour enfants en bas âge et adultes incontinents de l'invention comprend une feuille imperméable présentant une partie arrière et une partie avant. Un matelas absorbant est fixé sur la face interne de la feuille imperméable. La couche-culotte se complète par au moins un élément élastique de ceinture et deux dispositifs d'attache fixés dans la zone de la partie arrière de la feuille imperméable pour permettre de refermer la couche-culotte au moment de l'utilisation. Selon l'invention, la couche-culotte comprend une bande mince de ceinture réalisée en un matériau élastique, ladite bande étant disposée transversalement sur la partie arrière de la feuille imperméable en laissant subsister un bord extrême libre pour ladite partie arrière. La bande de ceinture est fixée uniquement dans la zone médiane de la feuille imperméable, à l'état non tendu, pendant la fabrication de la couche-culotte, entre la feuille imperméable et un élément de recouvrement formant un gousset pour la bande de ceinture. Les dispositifs d'attache sont fixés sur les extrémités de la bande de ceinture.

Grâce à cette position particulière des dispositifs d'attache et à l'existence d'un bord extrême libre sur la partie arrière de la couche-culotte, bord qui peut par exemple être rabattu afin d'assurer l'étanchéité, on obtient une couche-culotte qui peut être parfaitement ajustée de manière élastique au moment de la fermeture par les dispositifs d'attache. La fixation de la bande de ceinture uniquement dans la zone médiane de la partie arrière entraîne, à cet endroit, la formation de fronces qui ont l'effet d'une pince dans le dos, rattrapant ainsi le creux du dos et améliorane l'ajustement de la couche-culotte.

La bande de ceinture fixée à l'état non tendu reste libre à l'intérieur de son gousset et peut être saisie directement par les dispositifs d'attache fixés à ces deux extrémités, au moment de l'utilisation.

La bande de ceinture se trouve de préférence entre la feuille imperméable et la portion arrière du matelas absorbant. Le matelas absorbant est avantageusement fixé par collage, au moyen de lignes longitudinales d'un adhésif liquide à chaud posées sur la face interne de la feuille imperméable et sur la face opposée de l'élément de recouvrement. L'élément de recouvrement est de préférence fixé par collage sur la face interne de la feuille imperméable, de chaque côté de la bande de ceinture.

La bande de ceinture peut être fixée à la feuille imperméable par un matériau adhésif liquide déposé à chaud, par soudure réalisée au moyen de l'application d'ultrasons ou par calandrage.

La bande de ceinture peut avoir, à l'état non tendu, une longueur égale à la largeur de la partie arrière de la couche-culotte, des échancrures étant alors prévues dans la zone de bordure latérale de la couche-culotte. Les dispositifs d'attache dépassent légèrement à l'extérieur des échancrures.

Dans un autre mode de réalisation, la bande de ceinture est plus longue et dépasse donc de part et d'autre de la partie arrière de la couche-culotte. Les dispositifs d'attache sont également fixés sur les deux extrémités de la bande de ceinture.

La plupart du temps, la couche-culotte présente une forme analogue à un sablier, des échancrures latérales facilitant le passage des jambes. Toutefois, dans un autre mode de réalisation de l'invention, la feuille imperméable formant l'enveloppe extérieure de la couche-culotte ainsi que le matelas absorbant, présentent une forme générale rectangulaire allongée. Les portions extrêmes de la bande de ceinture dépassent les bords transversaux de la feuille imperméable et les deux dispositifs d'attache sont fixés au bout desdites portions extrêmes de la bande de ceinture. Dans un tel mode de réalisation, la bande de ceinture dépasse donc largement les bords de la couche-culotte et celle-ci n'est pas fermée à la ceinture pendant l'utilisation, une échancrure subsistant sur les côtés latéraux pour le passage des jambes et jusque sur les hanches. Il en résulte non seulement une économie de matière mais également une plus grande aisance pour l'utilisateur grâce en particulier à l'ajustement élastique réalisé par la ceinture élastique.

Les dispositifs d'attache peuvent être de type classique, comportant des éléments adhésifs repliés. On peut également utiliser d'autres types d'attaches tels que dispositifs à accrochage, à pression, etc.

Dans un mode de réalisation préféré, chaque dispositif d'attache comprend un élément de fixation plat, rendu solidaire par l'une de ses faces, au moyen d'une couche d'adhésif permanent, de l'extrémité de la bande de ceinture. L'autre face de l'élément de fixation comporte une couche d'adhésif destinée à entrer en contact avec la surface externe de la feuille imperméable lors de la fermeture de la couche-culotte.

Dans un autre mode de réalisation avantageux, chaque dispositif d'attache comprend un élément de fixation, lequel comporte une partie d'ancrage et une partie de fixation. La partie d'ancrage est rendue solidaire, au moyen d'une couche d'adhésif permanent de l'extrémité de la bande de ceinture. La partie de fixation est solidaire de la partie d'ancrage et repliée en double sur cette dernière. La partie de fixation comporte une zone munie d'une couche d'un matériau antiadhésion à l'intérieur du deuxième repli et une zone munie d'une couche d'adhésif en contact avec le matériau anti-adhésion. De cette manière, l'ensemble peut se déplier par pelage à la suite d'une traction sur l'extrémité de l'élément de fixation lors de la fermeture de la couche-culotte.

Un voile de non-tissé éventuellement hydrophobe tout en restant perméable à l'humidité, peut avantageusement être fixé par collage sur les zones de la feuille imperméable qui ne sont pas recouvertes par le matelas absorbant et sur la face interne du matelas absorbant. Des éléments élastiques d'entrejambe peuvent être prévus et sont de préférence disposés longitudinalement de part et d'autre du matelas absorbant au voisinage des bords latéraux dudit matelas.

La bande de ceinture peut être réalisée en tout matériau élastique approprié tel que le latex ou autre matériau qui peut être aisément fixé par collage et qui présente des caractéristiques élastiques désirées ainsi que es caractéristiques convenables de glissement à l'intérieur du gousset formé sur la partie arrière de la feuille imperméable. On peut également utiliser une matière textile tissée ou non et présentant les caractéristiques précédemment mentionnées, par exemple par l'adjonction de fils de latex ou de fibres élastiques. On peut également utiliser un matériau rétractable à chaud et capable, après rétraction, de présenter des caractéristiques d'élasticité.

La feuille imperméable dont il est question dans la présente invention est une feuille souple mince, réalisée dans un matériau imperméable aux liquides. Ce matériau peut, avantageusement, rester toutefois perméable aux gaz et en particulier à l'air. Ce voile de non-tissé qui peut être utilisé dans le cadre de l'invention peut, dans certains cas, présenter une certaine élasticité.

L'invention sera mieux comprise à l'étude de la description détaillée de quelques modes de réalisation décrits à titre d'exemples nullement limitatifs et illustrés par les dessins annexés sur lesquels:

la figure 1 est une vue schématique d'une couche-culotte selon l'invention représentée à l'état complètement étiré avec des arrachements partiels pour montrer la structure interne;

la figure 2 est une vue en coupe transversale selon II-II de la figure 1;

la figure 3 est une vue en coupe longitudinale selon III-III de la figure 1;

la figure 4 est une vue en coupe analogue à la figure 2 d'une variante de l'invention;

la figure 5 est une vue schématique montrant la couche-culotte refermée comme pour une utilisation;

la figure 6 est une vue schématique d'une variante d'une couche-culotte représentée comme dans le cas de la figure 1 en position complètement étirée et avec des arrachements partiels pour montrer la structure interne;

la figure 7 est une vue en coupe selon VII-VII de la figure 6;

la figure 8 est une vue montrant la couche-culotte fermée comme pour une utilisation normale;

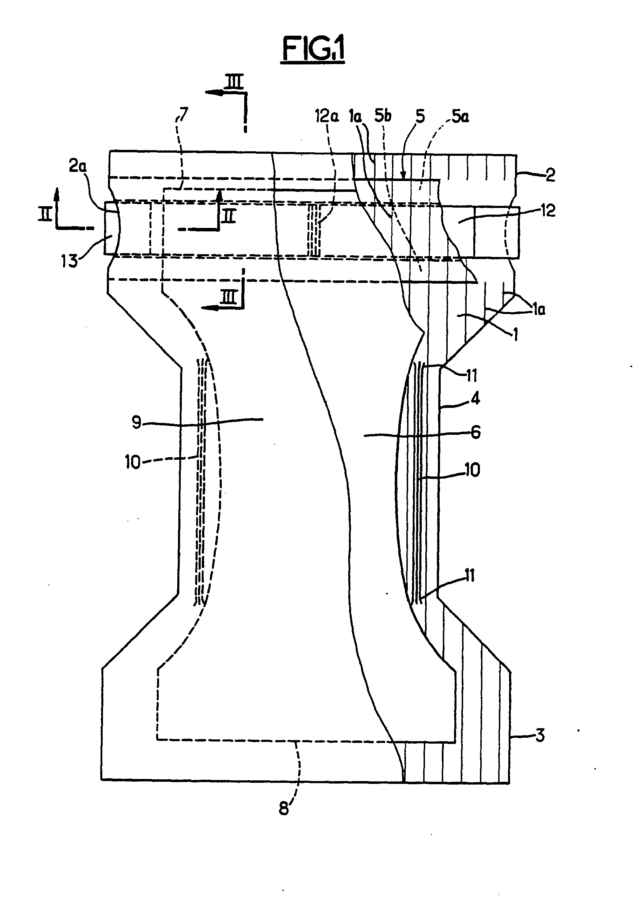

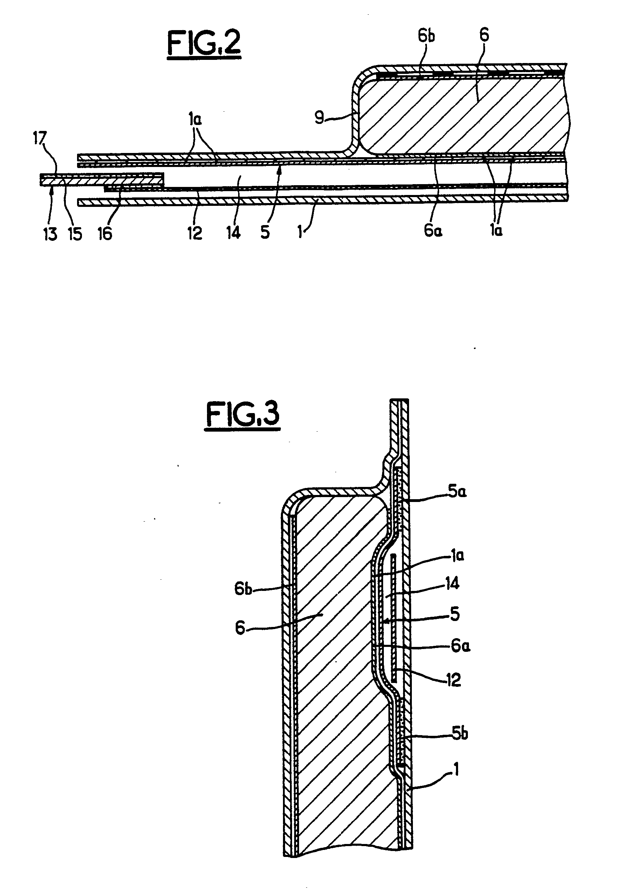

Tel qu'elle est illustrée sur les figures 1 à 3, une couche-culotte comprend une feuille mince imperméable 1 réalisée par exemple en polyéthylène, qui présente une partie arrière 2 et une partie avant 3 séparée par des échancrures latérales 4 permettant le passage des jambes. La forme générale qui en ré'sulte pour la couche-culotte est sensiblement celle d'un sablier. Un élément de recouvrement 5 est collé transversalement par deux bordures adhésives 5a et 5b sur la face interne de la feuille imperméable 1 à l'endroit de la partie arrière 2. Une pluralité de lignes longitudinales de collage la sont appliquées sur la face interne de la feuille 1 et sur la face de l'élément de recouvrement 5, opposée à la feuille 1, au moment de la fabrication, sous la forme d'un adhésif liquide qui se fige rapidement tout en gardant ses propriétés. Un matelas absorbant 6 affectant également une forme générale de sablier et présentant de ce fait une partie arrière 7 et une partie avant 8 est fixé sur la face interne de la feuille imperméable 1 et sur l'élément de recouvrement 5 au moyen des lignes longitudinales de collage la. Cette fixation peut se faire directement ou par l'intermédiaire d'un voile mince en ouate de cellulose 6a (figure 2). Un voile de non-tissé 9 vient recouvrir l'ensemble c'est-à-dire à la fois le matelas absorbant 6 recouvert d'un voile de cellulose 6b et, dans les zones latérales et extrêmes, la face interne de la feuille imperméable 1 ainsi que le bord extrême arrière de l'élément 5. Le voile de non-tissé 9 est également fixé par collage sur les portions latérales et extrêmes de la feuille 1 grâce à l'existence des lignes longitudinales la. Le matelas absorbant 6 peut de manière classique être réalisé en une ou plusieurs couches de ouate de cellulose défibrée éventuellement repliées ou posées les unes par rapport aux autres. Il peut avoir également subi un calandrage partiel améliorant la diffusion du liquide et la tenue de la ouate de cellulose. D'autres matériaux absorbants peuvent également être utilisés.

Des éléments élastiques latéraux 10 sont également posés longitudinalement de manière linéaire de chaque côté des bords du matelas absorbant 6 dans la zone de l'échancrure 4. Dans l'exemple illustré sur la figure 1, les éléments élastiques sont constitués par trois brins individuels réalisés par exemple en latex préalablement enduit d'un adhésif liquide à chaud permettant leur fixation par collage en continu sur la face interne de la feuille imperméable 1. Les extrémités 11 des brins élastiques 10 sont libres et se sont rétractées au moment de l'opération finale de découpe transversale de la couche-culotte. Pour obtenir ce résultat, l'enduction de l'adhésif sur ces élastiques est faite de manière intermittente selon un procédé classique. On comprendra bien entendu que d'autres types d'éléments élastiques pourraient être utilisés. En particulier, il serait possible d'utiliser un nombre de brins élastiques différent, par exemple 2, 4 brins ou plus. De plus on pourrait remplacer ces brins par d'autres éléments élastiques, par exemple sous forme de bandelettes minces. L'invention ne portant pas sur cette caractéristique de la couche-culotte, on comprendra en outre que les élastiques pourraient également être supprimés.

La couche-culotte se complète par une bande transversale de ceinture 12 qui est disposée dans la partie arrière 2 et collée uniquement sur la zone médiane 12a de ladite partie arrière sur la face interne de la feuille imperméable 1. Cette fixation est réalisée dans le mode de réalisation illustré sur la figure 1 par application d'un adhésif liquide à chaud. Cette fixation pourrait être réalisée différemment, par exemple par calandrage ou par ultrasons et d'une manière générale par toute méthode qui permettrait de fixer la bande de ceinture transversale 12 dans la zone médiane de la feuille imperméable 1.

Deux dispositifs d'attache 13 sont fixés sur les extrémités libres de la bande de ceinture transversale 12 de façon à dépasser légèrement du bord extrême de la partie arrière 2 lorsque la bande de ceinture est à l'état non tendu. Une échancrure 2a est pratiquée dans le bord de la partie arrière 2 de la feuille 1 et, au même endroit, sur le non-tissé 9 de façon à faciliter la préhension des dispositifs d'attache 13.

La bande de ceinture 12 est réalisée en un matériau élastique tel que le latex ou une matière textile élastifiée.

Elle peut également être réalisée en un matériau rétractable à chaud et capable après rétraction de présenter des caractéristiques d'élasticité. Des matériaux de ce type peuvent être obtenus par exemple à base de résine thermoplastique polyuréthane extrudée en couches minces qui sont ensuite étirées à chaud tout en étant maintenues à l'état étiré pendant le refroidissement. L'activation du matériau consiste à soumettre le matériau à un traitement thermique au cours duquel il reprend ses dimensions d'origine tout en conservant en quelque sorte mémorisées ses dimensions à l'état étiré. Dans ces conditions, le matériau présente des caractéristiques d'élasticité. La formation de ce matériau peut être faite par extrusion sous la forme de bande, de couche, de film, de pellicule, etc., suivie ensuite d'un étirage uniaxial entraînant une orientation moléculaire dans le matériau. A titre d'exemple de résine de polyuréthane thermoplastique qui peut être utilisée selon l'invention on peut citer des composés du type "Estane" fabriqué par B.F. GOODRICH, CO. La température d'orientation moléculaire de ces polyuréthanes varie selon leur structure entre 40 et 145°C. La rétraction ultérieure peut aller jusqu'à 50% des dimensions à l'état étiré. On pourra se reporter pour plus de précision au brevet U.S. n° 3 912 565.

De la même manière, on pourra se reporter au brevet U.S. n° 3 639 917 qui décrit d'autres matériaux rétractables à chaud constitués de blocs polymères élastomères de formule A-B-A où A est un bloc polymère non élastomère et B un bloc polymère élastomère. A est avantageusement un polymère dérivé d'un monomère tel que le styrène, le méthylstyrène ou le chlorostyrène, tandis que le polymère B est avantageusement un polymère du type polyisoprène élastomère.

Les matériaux préférés de ce type sont ceux décrits dans le brevet U.S. n° 4 303 571 auquel on pourra se référer pour plus de précisions. Ces matériaux sont constitués de mélanges de polymères élastomères thermoplastiques filmogènes, et plus particulièrement des mélanges constitués d'élastomères du type copolymère éthylènepropylène ou terpolymère éthylènepropylène-diène avec des résines copolymères thermoplastiques éthylène-acétate de vinyle. Le matériau extrudé sous la forme d'une bande mince est alors soumis comme précédemment à un étirage unidirectionnel à une température d'environ 65°C, la dimension dans le sens de la traction étant augmentée d'environ 400%. Après refroidissement, le polymère se trouve orienté uniaxialement et garde les dimensions étirées qu'il avait acquises à haute température. Il est donc possible de découper ce matériau de façon à réaliser des bandes qui peuvent être posées dans cet état de manière aisée pendant la fabrication en continu des couches-culottes. Un traitement ultérieur de chauffage sur toute la surface du matériau ou sur certaines zones entraîne, pour les zones chauffées à une température de l'ordre de 70°C, un retour du matériau à ses dimensions d'origine avant l'étirage à chaud. Par ailleurs, on constate que le matériau présente des caractéristiques élastiques et peut être étiré d'environ 50%. On peut également procéder au traitement de chauffage, entraînant le retour du matériau à son état d'origine, avant l'étape de pose de la bande de ceinture. La pose se fait alors dans les mêmes conditions qu'avec un matériau élastique de type classique.

On pourra mentionner encore à titre d'exemples de matériaux qu'il est possible d'utiliser dans l'invention, ceux décrits dans le brevet U S n° 3 819 401 et en particulier des dispersions colloïdales de polychlorure de vinyle dans des plastifiants tels que du diméthylphtalate.

La longueur totale de la bande de ceinture 12 est choisie, comme on peut le voir sur les figures 1 et 2, de façon qu'à l'état non tendu ou rétracté, avant utilisation, les deux dispositifs d'attache 13 fassent très légèrement saillie sur les bords de la partie arrière 2 à l'extérieur des échancrures 2a. On peut alors facilement saisir successivement chaque dispositif d'attache 13 et exercer une traction sur la moitié correspondante de la bande de ceinture 12 dont la partie médiane est fixée sur la zone 12a. La matière élastique de la bande 12 se tend librement à l'intérieur d'un gousset 14 (figures 2, 3) formé entre la feuille imperméable et l'élément de recouvrement 5, lequel n'est fixé que sur ses bords 5a et 5b.

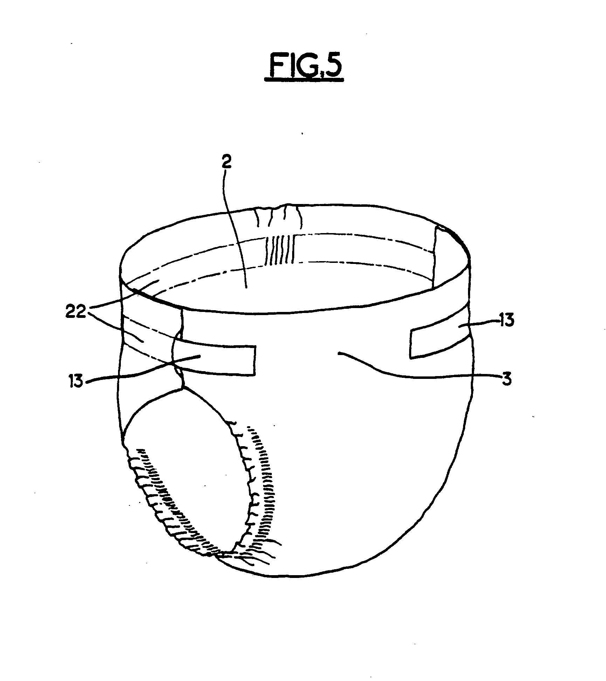

En se reportant principalement à la figure 2 on voit que le dispositif d'attache 13 comprend un élément de fixation 15 qui a la même largeur que la bande de ceinture 12 (figure 1) et qui est constitué par exemple par une pièce en papier, plate c'est-à-dire non repliée sur elle-même. Cet élément de fixation plat est rendu solidaire par une de ses faces de l'extrémité de la bande de ceinture 12 au moyen d'une couche d'adhésif permanent 16. L'élément de fixation 15 se trouve donc simplement dans le prolongement de la bande de ceinture 12. La face opposée de l'élément de fixation 15 est revêtue d'une couche d'adhésif 17 qui, dans l'exemple de réalisation illustré sur la figure 2, n'est pas recouverte par un élément de protection et est destinée à entrer en contact avec la surface externe de la feuille imperméable 1 lors de la fermeture de la couche-culotte, comme illustré en particulier sur la figure 5. Dans une variante, on peut prévoir de poser un élément de protection sur la couche adhésive 17, élément qui doit alors être retiré par pelage avant l'utilisation.

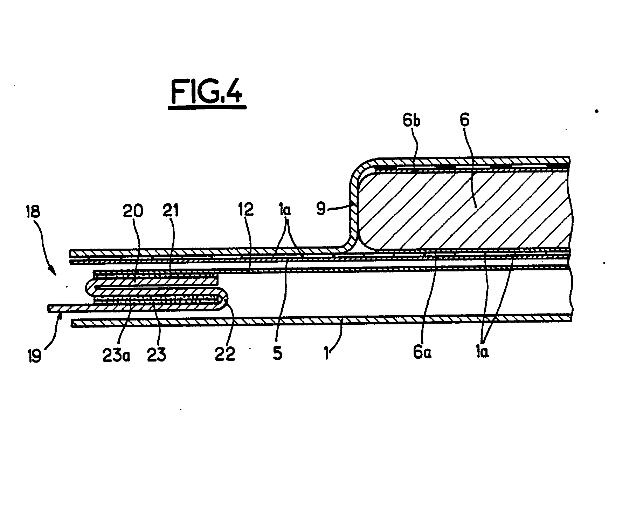

La figure 4 illustre une variante du dispositif d'attache qui peut être utilisé dans le cadre de la présente invention. Dans ce mode de réalisation, le dispositif d'attache 18 est réalisé sous la forme d'une structure à double repli. L'élément de fixation 19 réalisé par exemple sous la forme d'une pièce en papier, présente la même largeur que celle de la bande de ceinture 12. L'élément de fixation 19 comporte une partie d'ancrage 20 qui est rendue solidaire de l'extrémité de la bande de ceinture 12 au moyen d'une couche d'adhésif permanent 21. La partie de fixation proprement dite 22 fait suite à la partie d'ancrage 20 et est repliée en double sur cette dernière comme on peut le voir sur la figure 4. Dans le premier repli, les deux faces de la pièce de papier de l'élément 19 sont simplement en contact. Dans le deuxième repli, la partie de fixation 22 comporte une couche d'adhésif 23 qui est en contact avec une zone munie d'une couche 23a d'un matériau traité de façon à réduire l'adhérence. Lorsque l'on exerce une traction sur l'extrémité de l'élément de fixation 19, on provoque non seulement le dépliage du premier repli mais également le dépliage du deuxième repli avec pelage de la couche d'adhésif 23 par rapport à la couche anti-adhésive 23a. L'état final du dispositif d'attache 18 correspond alors à une extension maximale de l'élément de fixation 19, les deux replis étant supprimés. L'élément de fixation 19 est solidaire par la partie d'ancrage 20 de l'extrémité de la bande de ceinture 12 et peut être fixé par adhésion sur la surface extérieure de la feuille imperméable 1 lors de la fermeture de la couche-culotte grâce à la couche adhésive 23. Cette même traction provoque, comme dans le mode de réalisation précédent, l'extension du matériau élastique de la bande de ceinture 12 qui reste attachée par sa zone médiane 12a.

Comme on peut le voir sur la figure 5, la couche-culotte de l'invention peut être aisément ajustée au moment de l'utilisation grâce à une traction convenable sur les dispositifs d'attache 13 qui transmettent l'effort de traction directement à la bande de ceinture élastique 12 fixée dans la zone médiane de la partie arrière de la couche-culotte. Le bord extrême supérieur de la couche-culotte reste pratiquement exempt de tout fronçage. Il est donc possible d'assurer une étanchéité convenable au niveau de la ceinture par exemple en rabattant vers l'intérieur ce bord extrême de la partie arrière 2 ainsi que de la partie avant 3. A l'endroit de la fixation 12a de la bande de ceinture, les quelques fronces qui se trouvent formées peuvent en outre améliorer l'ajustement de la couche-culotte en rattrapant le creux du dos.

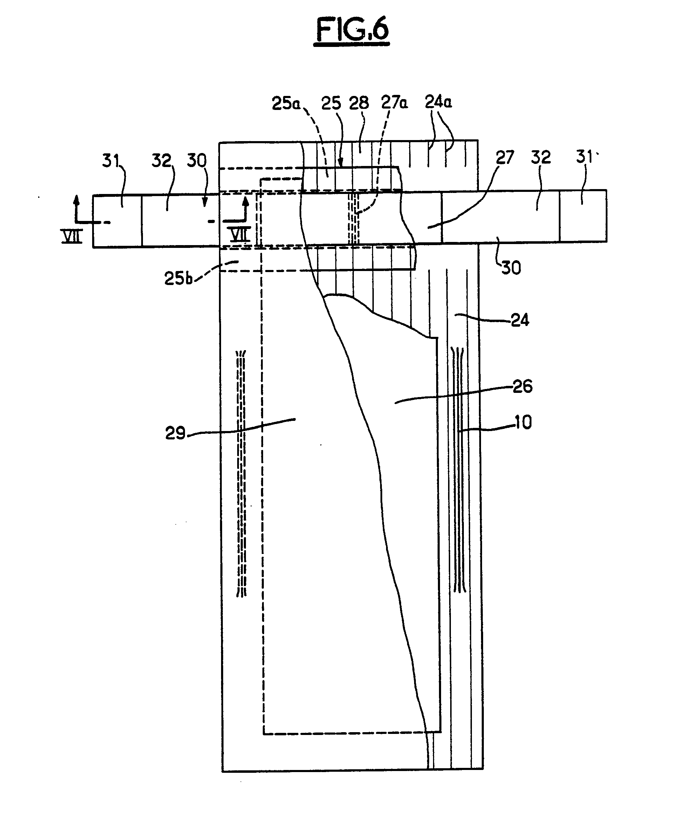

En se rapportant maintenant aux figures 6 à 8, on va décrire un deuxième mode de réalisation de l'invention. Dans ce mode de réalisation, la feuille imperméable 24 est de forme rectangulaire. Sa largeur est inférieure à la largeur totale des parties arrière et avant de la couche-culotte du mode de réalisation précédent. Un élément de recouvrement 25 est fixé sur toute la largeur de la feuille 24 par des bords adhésifs 25a et 25b. La feuille 24 est également munie de lignes longitudinales de collage 24a qui jouent le même rôle que les lignes 1a du mode de réalisation précédent. Le matelas absorbant 26 est de forme rectangulaire et il est fixé comme précédemment par les lignes de collage 24a sur la face interne de la feuille imperméable 24 et sur la surface de l'élément 25 qui est opposée à la feuille 24. Des éléments élastiques rectilignes 10 sont disposés comme précédemment au voisinage des bords extrêmes latéraux du matelas absorbant 26, sensiblement dans la zone médiane de ce dernier. Comme précédemment, ces éléments élastiques sont collés sur la zone médiane de la feuille 24.

Une bande élastique de ceinture 27 est placée transversalement au voisinage de l'un des petits côtés du rectangle formé par la feuille imperméable 24, en laissant subsister un bord extrême 28 qui présente sensiblement la même largeur dans le sens longitudinal que celle de la bande de ceinture 27. La bande 27 est collée sur la feuille 1 uniquement dans sa zone médiane 27a et elle peut se déplacer librement dans un gousset 27b formé entre l'élément de recouvrement 25 et la feuille 24. On notera que, comme précédemment, le matelas absorbant 26 vient recouvrir l'élément 25, son bord extrême étant fixé sur le bord 28 par les lignes de collage 24a. On pourrait envisager que le bord extrême du matelas absorbant 26 se trouve au droit de la bande de ceinture 27.

Un voile de non-tissé 29 vient recouvrir l'ensemble et est collé à la fois sur la face interne de la feuille 24 par les lignes de collage 24a et par l'intermédiaire d'un voile en ouate de cellulose 26b sur le matelas 26.

La bande de ceinture 27 dépasse largement de chaque côté de la feuille imperméable 24 et comporte donc deux extrémités latérales 30 s'étendant au-delà des bords latéraux de la feuille 24. Les bouts de chacune de ces extrémités 30 sont équipés de dispositifs d'attache par adhésif 31.

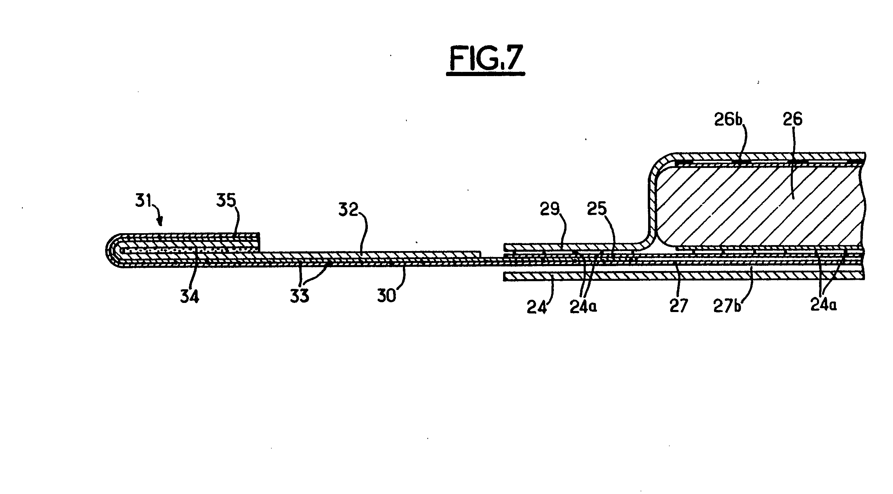

La vue en coupe de la figure 7 montre de manière plus précise la structure des extrémités 30 de la bande de ceinture 27. Les extrémités 30 sont revêtues du côté interne par rapport à la couche-culotte d'une pellicule en non-tissé ou en textile 32 qui peut être fixée par des lignes de collage 33 ou des soudures et qui s'étend jusqu'à l'intérieur du gousset 27b lorsque la bande 27 est à l'état non étiré. De cette manière, la surface de contact avec la peau de l'utilisateur est plus douce que dans le cas d'un contact direct avec le matériau de la bande de ceinture élastique 27 à l'état étiré. Dans l'exemple illustré sur la figure 7, le dispositif d'attache par adhésif 31 est intégré au bout de l'extrémité 30. Une zone 34 est revêtue d'une couche d'adhésif et le bout de l'extrémité 30 est replié sur l'adhésif 34 de façon à former l'élément de fixation 35 du dispositif d'attache par adhésif. Une traction sur l'élément de fixation 35 permet le pelage de la partie repliée qui peut alors venir se fixer sur la feuille imperméable 24 pour fermer la couche-culotte. Cette traction provoque également l'extension de la bande de ceinture 27 à l'intérieur du gousset 27b formé entre l'élément de recouvrement 25 et la feuille 24.

On notera que de nombreux autres types d'attache, que ce soit par adhésif ou par d'autres moyens, pourraient être utilisés.

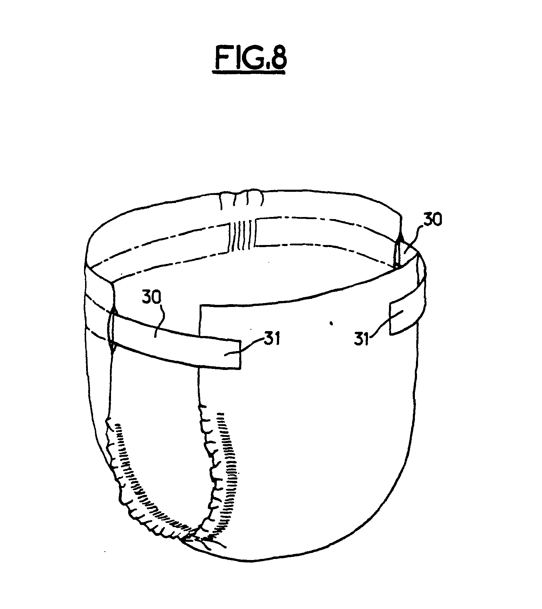

La figure 8 montre schématiquement une telle couche-culotte refermée comme pour une utilisation. On voit que dans ce mode de réalisation, le passage des jambes reste ouvert jusque sur les hanches. Comme précédemment, la zone arrière de la ceinture forme dans la zone médiane quelques fronces élastiques. La bande de ceinture élastique permet d'ajuster parfaitement la couche-culotte au moment de l'utilisation et au moment de la fermeture par les adhésifs 31.

**Revendications**

1. Couche-culotte à jeter pour enfants en bas âge et adultes incontinents, comprenant une feuille imperméable (1) présentant une partie arrière (2) et une partie avant (3), un matelas absorbant (6) fixé sur la face interne de la feuille imperméable, au moins un élément élastique de ceinture et deux dispositifs d'attache (13) fixés dans la zone de la partie arrière de la feuille imperméable pour permettre de refermer la couche-culotte au moment de l'utilisation, caractérisée par le fait qu'elle comprend une bande mince de ceinture (12) réalisée en un matériau élastique disposée transversalement sur la partie arrière (2) de la feuille imperméable (1) en laissant subsister un bord extrême libre de la partie arrière, la bande de ceinture avant été fixée, uniquement dans la zone médiane de la feuille imperméable à l'état non tendu, pendant la fabrication de la couche-culotte, entre la feuille imperméable et un élément de recouvrement formant un gousset pour la bande de ceinture; et que les dispositifs d'attache (13) sont fixés uniquement sur les extrémités libres de ladite bande de ceinture.

2. Couche-culotte selon la revendication 1, caractérisée par le fait que la longueur de la bande de ceinture à l'état non tendu est égale à la largeur de la partie arrière de la couche-culotte, des échancrures étant prévues dans la zone de bordure latérale de la couche-culotte, les dispositifs d'attache dépassant légèrement desdites échancrures.

3. Couche-culotte selon la revendication 1 ou 2, caractérisée par le fait que la bande de ceinture présente une longueur supérieure à la largeur de la partie arrière de la couche-culotte et dépasse de part et d'autres de ladite partie arrière, les dispositifs d'attache étant fixés au bout des deux extrémités de la bande de ceinture.

4. Couche-culotte selon la revendication 3, caractérisée par le fait que la feuille imperméable (24) ainsi que le matelas absorbant (26) présentent une forme générale rectangulaire allongée, une échancrure subsistant sur les côtés latéraux pour le passage des jambes et jusque sur les hanches lorsque la couche-culotte est fermée pour son utilisation.

5. Couche-culotte selon l'une quelconque des revendications précédentes, caractérisée par le fait que la bande de ceinture est réalisée en un matériau rétractable à chaud et capable après rétraction de présenter des caractéristiques d'élasticité.

6. Couche-culotte selon l'une quelconque des revendications précédentes, caractérisée par le fait que chaque dispositif d'attache comprend un élément de fixation plat rendu solidaire par l'une de ses faces par une couche d'adhésif permanent de l'extrémité de la bande de ceinture, l'autre face de l'élément de fixation comportant une couche d'adhésif destinée à entrer en contact avec la surface extérieure de la feuille imperméable lors de la fermeture de la couche-culotte.

7. Couche-culotte selon l'une quelconque des revendications 1 à 5, caractérisée par le fait que le dispositif d'attache comprend un élément de fixation comportant une partie d'ancrage rendue solidaire par une couche d'adhésif permanent de l'extrémité de la bande de ceinture; une partie de fixation solidaire de la partie d'ancrage et repliée en double sur cette dernière; une zone munie d'une couche d'un matériau anti-adhésion à l'intérieur du deuxième repli et une zone munie d'une couche d'adhésif en contact avec le matériau anti-adhésion; l'ensemble pouvant être déplié par pelage et traction sur l'extrémité de l'élément de fixation lors de la fermeture de la couche-culotte.

8. Couche-culotte selon l'une quelconque des revendications précédentes, caractérisée par le fait qu'un voile de non-tissé (9) perméable est fixé par collage sur les zones de la feuille imperméable non recouverte par le matelas absorbant.

9. Couche-culotte selon l'une quelconque des revendications précédentes, caractérisée par le fait que des éléments élastiques (10) sont disposés longitudinalement de part et d'autre du matelas absorbant au voisinage de ses bords latéraux.

10. Couche-culotte selon l'une quelconque des revendications précédentes caractérisée par le fait que le matelas absorbant est fixé par collage.

**Patentansprüche**

1. Wegwerfwindelhöschen für Kleinkinder und inkontinente Erwachsene, mit einer undurchlässigen Folie (1), die einen hinteren Teil (2) und einen Vorderteil (3) aufweist, einem saugfähigen Kissen (6), das auf der Innenseite der undurchlässigen Folie befestigt ist, wenigstens ein elastisches Taillenelement und mit zwei Verschlußmitteln (13), die im Bereich des hinteren Teils der undurchlässigen Folie befestigt sind, damit das Windelhöschen bei Gebrauch geschlossen werden kann, dadurch gekennzeichnet, daß es ein dünnes Taillenband (12) aufweist, das aus einem elastischen Werkstoff hergestellt ist, das quer auf dem hinteren Teil (2) der undurchlässigen Folie (1) angeordnet ist und einen freien Außenrand des hinteren Teils bestehen läßt, wobei das Taillenband bei der Herstellung des Windelhöschens ausschließlich im Mittelbereich der undurchlässigen Folie im nicht gespannten Zustand zwischen der undurchlässigen Folie und einem Abdeckelement, das eine Tasche für das Taillenband bildet, befestigt worden ist, und daß die Verschlußmittel (13) ausschließlich an den freien Enden des genannten Taillenbandes befestigt sind.

2. Windelhöschen nach Anspruch 1, dadurch gekennzeichnet, daß die Länge des Taillenbandes im nicht gespannten Zustand gleich der Breite des hinteren Teils des Windelhöschens ist, wobei im seitlichen Randbereich des Windelhöschens Ausschnitte vorgesehen sind und wobei die Verschlußmittel über diese Ausschnitte geringfügig vorstehen.

3. Windelhöschen nach Anspruch 1 oder 2, dadurch gekennzeichnet, daß das Taillenband eine Länge aufweist, die größer ist als die Breite des hinteren Teils des Windelhöschens, und auf beiden Seiten über den genannten hinteren Teil Vorsteht, wobei die Verschlußmittel am Ende der beiden Endbereiche des Taillenbandes befestigt sind.

4. Windelhöschen nach Anspruch 3, dadurch gekennzeichnet, daß die undurchlässige Folie (24) und auch das saugfähige Kissen (26) eine im wesentlichen länglich rechteckförmige Form besitzen, wobei an den Seiten eine bei für seine Verwendung geschlossenem Windelhöschen bis zu den Hüften reichende Ausnehmung für den Durchtritt der Beine vorgesehen ist.

5. Windelhöschen nach einem der vorhergehenden Ansprüche, dadurch gekennzeichnet, daß das Taillenband aus einem warmschrumpffähigen Werkstoff hergestellt ist, der nach dem Schrumpfen elastische Eigenschaften aufweisen kann.

6. Windelhöschen nach einem der vorhergehenden Ansprüche, dadurch gekennzeichnet, daß jedes Verschlußmittel ein flaches Befestigungselement aufweist, das mit einer seiner Seiten durch eine Dauerklebstoffschichte mit dem Ende des Taillenbandes verbunden ist, wogegen die andere Seite des Befestigungselementes eine Klebstoffschichte aufweist, die dazu bestimmt ist, beim Schließen des Windelhöschens mit der äußeren Fläche der undurchlässigen Folie in Berührung zu treten.

7. Windelhöschen nach einem der Ansprüche 1 bis 5, dadurch gekennzeichnet, daß das Verschlußmittel ein Befestigungselement, das einen Verankerungsteil aufweist, der über eine Dauerklebstoffschichte mit dem Ende des Taillenbandes verbunden ist, besitzt; einen Befestigungsteil, der mit dem Verankerungsteil verbunden ist und auf letzteren doppelt umgeschlagen ist; einen Abschnitt, der im Inneren der zweiten Umfaltung mit einem Antihaftwerkstoff beschichtet ist und einen Abschnitt aufweist, der mit einer Klebstoffschicht, die mit dem Antihaftstoff in Berührung steht, versehen ist; wobei die gesamte Anordnung durch Ablösen und Ziehen am Ende des Befestigungsmittels beim Schließen des Windelhöschens aufgefaltet werden kann.

8. Windelhöschen nach einem der vorhergehenden Ansprüche, dadurch gekennzeichnet, daß ein durchlässiges Vliestuch (9) durch Kleben auf den vom saugfähigen Kissen nicht bedeckten Bereichen der undurchlässigen Folie befestigt ist.

9. Windelhöschen nach einem der vorhergehenden Ansprüche, dadurch gekennzeichnet, daß beiderseits des saugfähigen Kissens in der Nähe seiner Seitenränder in Längsrichtung verlaufende elastische Elemente (10) angeordnet sind.

10. Windelhöschen nach einem der vorhergehenden Ansprüche, dadurch gekennzeichnet, daß das saugfähige Kissen durch Kleben befestigt ist.

**Claims**

1. A disposable nappy for infants or incontinent adults, comprising an impermeable sheet (1) having a rear portion (2) and a front portion (3), an absorbent pad (6) fixed to the inner side of the impermeable sheet, at least one elastic waist element and two fastening devices (13) fixed in the zone of the rear portion of the impermeable sheet to make it possible to close up the nappy at the time of use, characterized in that it comprises a thin waist band (12) made of an elastic material disposed transversely to the rear portion (2) of the impermeable sheet (1) allowing an end edge of the rear portion to remain free, the waist band having been fixed solely in the median zone of the impermeable sheet in its unstretched state during the manufacture of the nappy between the impermeable sheet and a covering element forming a pocket for the waist band; and in that the fastening devices (13) are fixed solely to the free ends of the said waist band.

2. A nappy according to claim 1, characterized in that the length of the waist band in its unstretched state is equal to the width of the rear portion of the nappy, provision being made for openings in the lateral edge zone of the nappy, the fastening devices extending slightly beyond the said openings.

3. A nappy according to claim 1 or 2, characterized in that the waist band has a length exceeding the width of the rear portion of the nappy and projects on either side from the said rear portion, the fastening devices being fixed to the ends of the two end portions of the waist band.

4. A nappy according to claim 3, characterized in that the impermeable sheet (24), as well as the absorbent pad (26) have an elongate generally rectangular shape, an opening remaining on the lateral sides for the passing of the legs and as far as onto the hips when the nappy is fastened for use.

5. A nappy according to any one of the preceding claims, characterized in that the waist band is made of a heat-shrinkable material and capable, after shrinking, of showing characteristics of elasticity.

6. A nappy according to any one of the preceding claims, characterized in that each fastening device comprises a flat fixing element fixed on one of its sides to the end of the waist band by a permanent adhesive layer, the other side of the fixing element comprising an adhesive layer intended to come into contact with the outer surface of the impermeable sheet during the fastening of the nappy.

7. A nappy according to any one of claims 1 to 5, characterized in that the fastening device comprises a fixing element comprising an anchorage portion fixed by a permanent adhesive layer to the end of the waist band; a fixing portion integral with the anchorage portion folded to double up onto this latter; a zone provided with a layer of a non-adhesive material inside the second fold and a zone provided with an adhesive layer in contact with the non-adhesive material; it being possible for the whole to be unfolded by peeling off and pulling on the end of the fixing element during the fastening of the nappy.

8. A nappy according to any one of the preceding claims, characterized in that a permeable nonwoven cover (9) is fixed by glueing to the zones of the impermeable sheet that are not covered by the absorbent pad.

9. A nappy according to any one of the preceding claims, characterized in that elastic elements (10) are disposed longitudinally on either side of the absorbent pad near its lateral edges.

10. A nappy according to any one of the preceding claims, characterized in that the absorbent pad is fixed by glueing.

# FIG.1

## FIG.2

## FIG.3

# FIG.4

# FIG.5

# FIG.6

# FIG.7

# FIG.8